# FASCICULE DE BREVET EUROPEEN

(11) **EP 1 497 836 B1**
(45) Date de publication et mention de la délivrance du brevet: **04.03.2009**
(21) Numéro de dépôt: 03746353.6
(22) Date de dépôt: 17.04.2003
(51) Int. Cl.: G21F 3/00, A61B 6/10

(54) **PARAVENT DE PROTECTION CONTRE LES EMISSIONS DE RAYONNEMENTS IONISANTS**
SCHIRM ZUM SCHUTZ VOR EMISSIONEN IONISIERENDER STRAHLUNG
SCREEN FOR PROTECTION AGAINST IONISING RADIATION EMISSIONS

(30) Priorité: 17.04.2002 FR 0204768
(43) Date de publication de la demande: 19.01.2005
(73) Titulaire: LEMER PROTECTION ANTI-X PAR ABREVIATION SOCIETE LEMER PAX, 44470 Carquefou (FR)
(72) Inventeur: LEMER, Pierre-Marie, F-44100 Nantes (FR)
(74) Mandataire: Michelet, Alain
(86) Numéro de dépôt international: PCT/FR2003/001247
(87) Numéro de publication internationale: WO 2003/088267

(56) Documents cités:
- EP-A- 0 345 548
- WO-A-01/84558
- US-A- 3 308 297
- US-A- 4 581 538
- US-B1- 6 278 125

## Description

La présente invention concerne un paravent radioprotecteur, et plus particulièrement un paravent utilisé en milieu médical ou autre pour protéger un opérateur contre les émissions de rayonnements ionisants, par exemple les rayons X ou gamma.

Pour certaines interventions sur des patients, comme des examens du genre cathétérisme, pose de pacemaker, examens vasculaires, neurologiques ou urologiques... l'opérateur (technicien, médecin, chirurgien ou autre) doit être protégé contre les rayonnements ionisants auxquels le patient est soumis.

Les structures de protection existantes consistent en des vêtements du genre blouses, chasubles ou tabliers en matériau radioprotecteur.

Il existe aussi des écrans ou paravents constitués de panneaux ou d'assemblage de panneaux en matériau approprié posés verticalement directement sur le sol ou par l'intermédiaire d'une embase support.

Mais les vêtements en matériau radioprotecteur n'assurent pas une protection optimale pour l'opérateur, du fait en particulier qu'ils ne recouvrent pas la totalité du corps (tête, jambes, bras et pieds), et également du fait des contraintes de poids auxquels ces vêtements sont soumis. D'autre part, les écrans ou paravents radioprotecteurs actuels, par exemple tels que décrits dans les documents US-A-3 308 297 ou EP-A-0 345 548, ne sont pas adaptés pour permettre à un opérateur de travailler confortablement et en toute sécurité.

La présente invention propose une nouvelle structure de paravent radioprotecteur qui est particulièrement efficace et intéressante pour l'opérateur sur un plan ergonomique.

L'objet de l'invention procure d'une part une meilleure visibilité pour l'opérateur, et d'autre part un plus grand confort au niveau de son positionnement derrière le paravent, dans le cadre de son intervention. Cet opérateur bénéficie ainsi de meilleures conditions de travail, sans port de vêtement lourd, ce qui lui permet d'intervenir avec une plus grande précision et une meilleure efficacité, et ceci en toute sécurité.

Le paravent radioprotecteur selon la présente invention est constitué d'une paroi frontale, associée à une paroi latérale qui s'étend à l'équerre ou sensiblement à l'équerre à partir de l'un des côtés de ladite paroi frontale, et ces deux parois comportent des panneaux transparents sur une partie au moins de leur hauteur. La partie supérieure de la paroi frontale est inclinée vers l'avant, formant surplomb, pour permettre à l'opérateur de se rapprocher de la zone d'intervention, et elle est munie de deux orifices pour le passage des bras dudit opérateur.

Toujours selon l'invention, la paroi frontale du paravent est constituée d'un panneau inférieur vertical ou sensiblement vertical, prolongé par un panneau supérieur dont une partie au moins est réalisée en matériau transparent, lequel panneau supérieur est incliné vers l'avant, faisant un angle compris entre 10 et 30° par rapport à la verticale, et la paroi latérale épouse la forme en dièdre de ladite paroi frontale. De préférence, l'angle correspondant est compris entre 15 et 20° par rapport à la verticale.

Selon une forme de réalisation particulière, la paroi frontale est constituée d'un panneau inférieur en matériau opaque qui s'étend sur une hauteur comprise entre 60 et 100 cm, prolongé par un panneau supérieur qui s'étend jusqu'à un niveau correspondant au moins à la taille de l'opérateur, c'est-à-dire de l'ordre de 2 m.

Selon une autre particularité, l'un au moins des orifices de passage des bras de l'opérateur est doté d'une manchette en matériau radioprotecteur destinée à venir se serrer sur le poignet ou l'avant-bras de l'opérateur en vue d'optimiser la protection. Cette manchette se présente avantageusement sous la forme d'un « iris », constitué de lamelles souples montées sur une couronne circulaire. Cette couronne est associable au rebord dudit orifice par tout moyen approprié, genre bouton-pression par exemple , et les lamelles, au nombre de quatre par exemple, en matériau du genre caoutchouc plombé, se chevauchent et sont de préférence maintenues par un bracelet souple qui est situé à l'extérieur, à proximité de l'embouchure externe de la manchette.

Toujours selon l'invention, l'un des orifices de passage de bras est situé à proximité de l'angle formé par les parois frontales et latérales, et l'autre orifice est situé sur le bord libre de ladite paroi frontale, au même niveau que le précédent et ouvert latéralement de manière à faciliter le mouvement du bras correspondant.

Selon une première forme de réalisation possible, le panneau frontal supérieur muni des orifices de passage des bras comporte un système de doublage constitué d'un panneau mobile. Ce panneau mobile est muni lui-même d'orifices de passage des bras, en correspondance avec les orifices de la paroi frontale ; ces derniers orifices, de forme oblongue et surdimensionnés par rapport aux orifices dudit panneau mobile, s'étendent sur toute la surface balayée par lesdits orifices dudit panneau de doublage. Cette particularité permet un réglage de la hauteur des orifices de passage des bras de l'opérateur. De préférence, le panneau mobile de doublage est guidé sur la paroi frontale au moyen de glissières disposées latéralement. Ce panneau mobile est d'autre part verrouillable sur la paroi frontale, selon plusieurs positions adaptées au gabarit de l'opérateur, au moyen d'un doigt d'ancrage coopérant avec un index aménagé sur la structure de ladite paroi frontale.

Selon une autre forme de réalisation possible, et toujours pour permettre un réglage de la hauteur des orifices de passage des bras, la paroi frontale et la paroi latérale forment un ensemble monté coulissant verticalement sur un bâti ou soubassement muni de roulettes.

Le paravent comporte alors avantageusement un système de manoeuvre de l'ensemble constitué par la paroi frontale et la paroi latérale, en forme de vérin(s) piloté(s) par un organe de commande du genre pédale ou bouton-poussoir par exemple.

Toujours selon l'invention, la partie inférieure du paravent se présente sous la forme d'un bâti ou soubassement muni de roulettes installées au niveau des différentes arêtes avec, en plus, au moins une roulette supplémentaire disposée en saillie sur la face avant de la paroi frontale, portée par une console, permettant d'accroître le périmètre de sustentation dudit paravent, et ainsi sa stabilité.

Selon une autre disposition de l'invention, le paravent comporte, fixées sur les parois frontale et latérale, ou sur le châssis ou soubassement, des lamelles souples en matériau radioprotecteur, du genre caoutchouc plombé par exemple, permettant notamment le passage de pédales, câbles ou autres accessoires liés au matériel nécessaire à certains types d'interventions médicales ou autres.

Selon encore une autre disposition, le paravent comporte, sur les faces externes et internes de la paroi frontale, des barrettes ou des profilés permettant l'installation de champs stériles, aménagés sous le niveau des orifices de passage des bras.

Selon encore une autre disposition de l'invention, une paroi complémentaire en matériau radioprotecteur faisant office de plafond, s'étend au moins pour partie entre la paroi frontale et la paroi latérale du paravent.

Selon encore d'autres particularités, le paravent conforme à l'invention comporte un rideau souple de protection du dos de l'opérateur, ainsi qu'un bras d'appui amovible de soutien dudit opérateur.

Mais l'invention sera encore illustrée, sans être aucunement limitée, par la description suivante de deux modes de réalisation particuliers, donnés uniquement à titre d'exemples et représentés sur les dessins annexés dans lesquels :
- la figure 1 représente, en perspective et vue de trois-quarts avant, une première forme de réalisation possible d'un paravent radioprotecteur conforme à la présente invention ;
- la figure 2 représente le paravent de la figure 1, toujours en perspective, montrant sa partie intérieure ;
- la figure 3 représente, vue d'une façon agrandie, une manchette protectrice en forme d'iris, adaptable au niveau de l'orifice circulaire de passage d'un bras, et en particulier du bras gauche de l'opérateur dans l'exemple de paravent représenté sur les figures 1 et2;
- la figure 4 est une vue de face d'une seconde forme de réalisation possible d'un paravent radioprotecteur conforme à l'invention ;
- la figure 5 montre le paravent de la figure 4, en perspective et vu de trois-quarts avant ;
- la figure 6 est une vue en perspective, vu de trois-quarts arrière, du paravent illustré sur les figures 4 et 5.

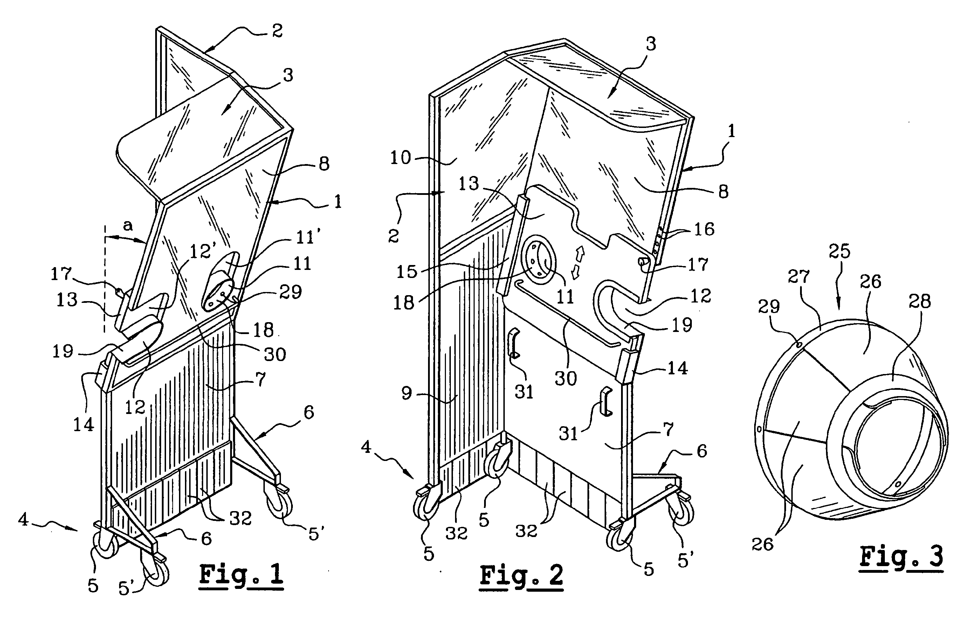

Tel que représenté sur les figures 1 et 2, le paravent radioprotecteur comprend une paroi frontale 1 et, disposée à l'équerre ou sensiblement à l'équerre, une paroi latérale 2.

La partie supérieure du paravent comporte une paroi supplémentaire faisant office de plafond 3, qui peut s'étendre sur tout ou partie de l'espace situé entre les rebords supérieurs des parois frontale 1 et latérale 2.

Ce genre de paravent, destiné à protéger un opérateur contre des émissions de rayonnements ionisants, est constitué de panneaux en matériau radioprotecteur approprié. Les différents panneaux sont portés par une ossature métallique, par exemple en aluminium, qui intègre un blindage permettant une continuité de radioprotection au niveau de toutes les jonctions.

Ce paravent comprend des panneaux inférieurs qui sont opaques (par exemple en panneaux de bois blindés de feuilles de plomb), et des panneaux supérieurs qui sont transparents (par exemple en verre au plomb ou plexiglas plombé) pour offrir une visibilité frontale et latérale à l'opérateur qui intervient notamment en milieu médical, pour des opérations où le patient est soumis à des rayonnements.

Le soubassement 4 du paravent est muni de roulettes 5 qui permettent son déplacement de manière aisée. On remarque que des roulettes 5 sont disposées au niveau de chaque arête des parois latérale et frontale ; d'autre part, des roulettes 5' complémentaires sont disposées devant la paroi frontale 1, portées par des consoles 6 solidaires de cette dernière, de façon à augmenter la surface du périmètre de sustentation du paravent, et donc sa stabilité. Les différentes roulettes 5, 5' sont de préférence pivotantes et munies d'un système de frein déverrouillable.

La paroi frontale 1 comprend une partie inférieure 7 constituée d'un panneau opaque vertical, et d'une partie supérieure 8 constituée d'un panneau transparent.
Ce panneau transparent 8 est incliné vers l'avant, c'est-à-dire vers le champ opératoire. Son inclinaison, d'un angle a compris entre 10 et 30° par rapport à la verticale, et de préférence compris entre 15 et 20°, permet à l'opérateur de se pencher vers l'avant au moment de son intervention, et ainsi de se rapprocher de la zone opératoire, pour une meilleure visibilité et un plus grand confort, notamment.

La paroi latérale 2 s'étend verticalement sur le côté ; elle comporte elle aussi un panneau inférieur opaque 9 et un panneau supérieur transparent 10 qui procure une visibilité latérale à l'opérateur, en particulier pour surveiller son patient.

Cette paroi latérale 2 épouse la forme en dièdre de la paroi frontale 1 ; le panneau transparent 10 a une forme sensiblement trapézoïdale.

La paroi de plafond 3 est ici réalisée en matériau transparent, mais elle pourrait de la même façon être obtenue en matériau opaque si la visibilité supérieure ne présente pas d'intérêt.

Les dimensions des différentes parois sont choisies de façon à permettre l'accueil de tous les gabarits d'opérateurs. Ainsi, la paroi frontale 1 et la paroi latérale 2 peuvent avoir une hauteur de l'ordre de 2 m par exemple.

Le panneau inférieur 7 de la paroi frontale 1 s'étend jusqu'à un niveau qui correspond par exemple au niveau de la table d'opération ; ce panneau inférieur peut s'étendre sur une hauteur comprise entre 60 et 100 cm, de préférence voisine de 80 cm.

Le panneau supérieur transparent 8 de la paroi frontale 1 s'étend donc entre le niveau de la table d'opération, c'est-à-dire environ 80 cm, et une hauteur de l'ordre de 2 m. Il comporte dans sa partie inférieure, c'est-à-dire au-dessus du niveau de la table d'opération, des orifices 11 et 12 de passage des bras de l'opérateur, pour permettre à ce dernier d'intervenir sur un patient, et ceci de manière pratique et sûre.

Des moyens particuliers sont prévus sur ce paravent radioprotecteur pour permettre de déplacer verticalement les orifices 11 et 12 de passage de bras, de manière à pouvoir adapter leur niveau selon la taille de l'opérateur.

Ainsi, ces orifices 11 et 12 sont aménagés, d'une part dans le panneau 8 de la paroi frontale 1, et d'autre part dans un panneau de doublage 13 aménagé à l'intérieur du paravent. Ce panneau de doublage interne 13 est aménagé sur la face interne du panneau supérieur 8 de la paroi frontale 1 ; il est prévu mobile parallèlement audit panneau 8, guidé dans des glissières latérales 14 et 15 solidaires de la paroi frontale 1, et sa position est établie au moyen d'un index 16 coopérant avec un doigt d'ancrage 17. Le système d'indexage 16 est constitué de plusieurs orifices espacés verticalement sur le rebord latéral du panneau 8 ; le doigt d'ancrage 17 est solidaire du panneau mobile 13 et il est agencé pour s'engager dans l'un des orifices de l'index 16, en fonction du niveau de positionnement désiré des orifices 11 et 12 de passage des bras.

Les orifices 11 et 12 aménagés dans le panneau mobile 13 sont avantageusement équipés de bagues 18, 19, démontables et facilement autoclavables, fixées au moyen de vis captives.

Le panneau 8 de la paroi frontale 1 comporte des orifices 11' et 12' de forme oblongue, surdimensionnés par rapport aux orifices 11 et 12 du panneau mobile 13, et dont les dimensions, formes et positionnements sont adaptés à la course dudit panneau de doublage 13. Ces orifices 11' et 12' restent masqués en permanence par le panneau de doublage 13, quel que soit le positionnement de ce dernier.

Le panneau de doublage 13 est par exemple réalisé avec le même matériau radioprotecteur que le panneau supérieur 8 de la paroi frontale 1.

L'orifice 11 situé dans le panneau mobile 13 est un orifice circulaire ; il est situé à proximité de l'angle formé par les parois frontale 1 et latérale 2. Cet orifice circulaire 11 est adapté pour le passage du bras gauche de l'opérateur, pour le mode de réalisation représenté sur les figures 1 et 2.

L'orifice 12 aménagé dans le panneau mobile 13 est disposé vers le rebord libre de la paroi frontale 1, et il est ouvert latéralement, se présentant en fait en forme de U couché pour permettre au bras correspondant de l'opérateur de se dégager facilement et de conserver une grande liberté de mouvement.

Pour augmenter la protection de l'opérateur, on prévoit de limiter le passage des rayonnements ionisants par l'orifice 11 en équipant ce dernier d'une manchette de protection 25 en forme d' « iris ». Cet iris 25 est représenté isolément sur la figure 3. Il est constitué d'un assemblage de lamelles 26, ici au nombre de quatre, qui se chevauchent partiellement et qui sont fixées sur une couronne circulaire 27 dont le diamètre correspond sensiblement au diamètre de l'orifice 11.
Ces lamelles 26 sont par exemple réalisées en matériau du genre caoutchouc plombé; elles sont de préférence retenues élastiquement au niveau de leur embouchure, au moyen d'un bracelet 28.

La manchette 25 en forme d'iris est fixée dans l'orifice 11, sur la bague 18, par l'intermédiaire de sa couronne circulaire 27, au moyen d'un système de boutons-pressions 29 par exemple.

Sur les figures 1 et 2, on remarque que le paravent conforme à l'invention est équipé de barrettes ou profilés 30, d'une part sur le panneau de doublage 13, et d'autre part sur la face externe du panneau 8, destinés notamment à la fixation de champs stériles. Sur la face interne du panneau 7, on remarque également la présence de deux poignées de manutention 31.

A sa partie inférieure, c'est-à-dire entre le bâti ou soubassement 4 sur lequel sont fixées les roulettes 5, 5' et le sol, le paravent comporte un tablier souple de protection constitué d'une juxtaposition de lamelles 32. Ces lamelles 32 peuvent se superposer partiellement ; elles sont réalisées en caoutchouc chargé de plomb, par exemple, et elles permettent le passage d'accessoires du genre câbles ou pédales de commande utiles pour certains types d'intervention.
Ces lamelles 32 sont fixées par tout moyen approprié à la partie inférieure des panneaux 7 et 9 des parois 1 et 2 respectivement.

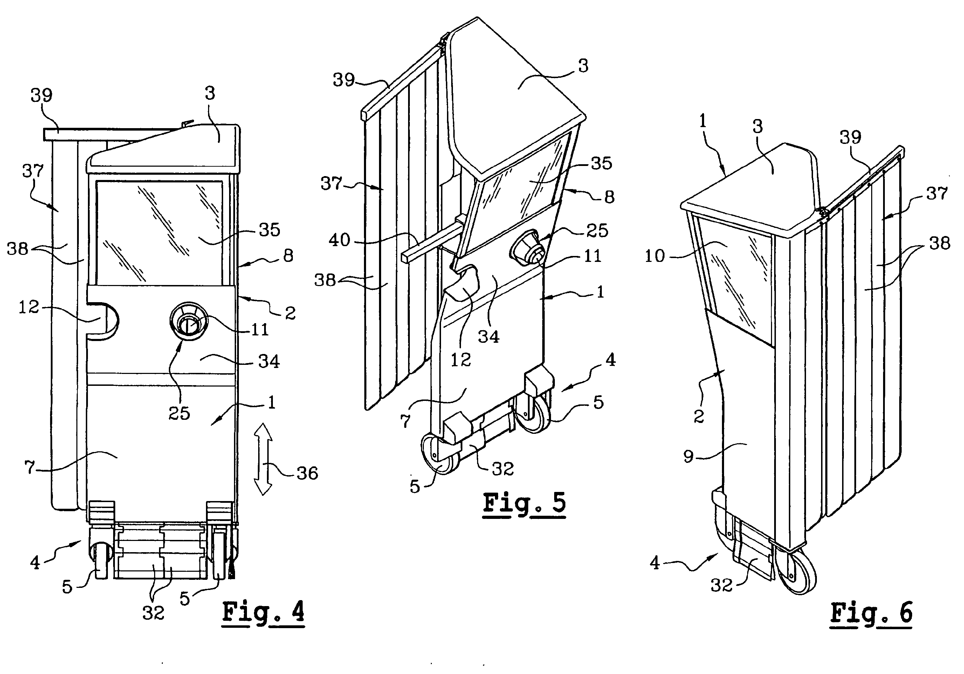

Les figures 4 à 6 montrent une variante de réalisation d'un paravent radioprotecteur conforme à l'invention.
Dans cette variante de réalisation, les parties communes au mode de réalisation précédent conservent les mêmes repères pour faciliter la compréhension.

On retrouve donc la paroi frontale 1, la paroi latérale 2 et la paroi de plafond 3 portées par un bâti ou soubassement 4 muni de roulettes 5.

La paroi frontale 1 comprend une partie inférieure 7 constituée d'un panneau opaque vertical, et une partie supérieure 8 inclinée vers l'avant d'un angle compris entre 10 et 30° (et de préférence entre 15 et 20°) par rapport à la verticale. Cette partie supérieure 8 comprend une zone opaque 34 située dans le prolongement du panneau inférieur 7, munie des deux orifices 11 et 12 de passage des bras de l'opérateur ; cette zone opaque 34 est surmontée d'une zone transparente 35.
L'orifice 11 est circulaire et il est associé à une manchette de protection 25 en saillie vers l'extérieur ; l'orifice 12 est en forme de U couché, ouvert latéralement. Comme pour le mode de réalisation décrit précédemment, les orifices 11 et 12 sont avantageusement équipés de bagues démontables.

La paroi latérale 2 s'étend verticalement et elle comporte une partie inférieure opaque 9 surmontée d'une partie supérieure transparente 10. Dans ce mode de réalisation, la paroi de plafond 3 est opaque.

Les parois frontale 1, latérale 2 et le plafond 3 sont portés par une ossature métallique en aluminium blindé.

Cette ossature métallique peut être réglable en hauteur par rapport au soubassement porteur 4, de manière à pouvoir adapter le niveau des orifices 11 et 12 selon la taille de l'opérateur. Cette possibilité de réglage, illustrée par la double flèche 36 de la figure 4, est obtenue par un montage coulissant de l'ossature métallique en question sur le soubassement 4. Ce montage coulissant peut par exemple être réalisé au moyen de coulisses de guidage solidaires du soubassement, venant s'intégrer dans les montants verticaux de l'ossature métallique ; un ou plusieurs vérins, de type hydrauliques ou autres, constituent le système de manoeuvre du mouvement, piloté(s) par un organe de commande du genre pédale, bouton-poussoir, manette ou autre.
Le système de réglage correspondant n'apparaît pas sur les figures.

Tel qu'on peut le voir sur les figures 4 à 6, le paravent conforme à l'invention comporte un rideau souple 37 permettant la protection du dos de l'opérateur. Ce rideau souple 37 est avantageusement constitué d'une juxtaposition de lamelles souples 38 en caoutchouc plombé, montées sur un bras support 39 fixé en porte-à-faux à la partie supérieure du paravent, par exemple sur la bordure libre de la paroi de plafond 3, ou sur la bordure supérieure de la paroi latérale 2.
De préférence, le bras support 39 est monté articulé autour d'un axe vertical pour permettre la mise en place et le retrait du rideau, ou simplement pour régler son positionnement derrière l'opérateur.
Ce rideau souple 37 constitue une sorte de paroi mobile complétant efficacement la radioprotection.

Sur la figure 5, on remarque également la présence d'un bras 40 qui s'étend horizontalement, en porte-à-faux à partir de la paroi latérale 2, sensiblement à mi-hauteur du paravent, destiné à servir d'organe d'appui pour le dos ou les reins de l'opérateur.
Ce bras d'appui et de soutien 40 est de préférence amovible ; il peut être monté articulé sur la paroi latérale 2, associé à une tringle de contreventement escamotable.

Sur les figures 4 à 6, on remarque aussi la présence des lamelles 32 en caoutchouc plombé complétant la protection en partie inférieure, dans le prolongement des parois frontale 1 et latérale 2. Ces lamelles de protection 32 peuvent être fixées sur le soubassement 4 et/ou sur la bordure inférieure des parois frontale 1 et latérale 2.

Les différents accessoires décrits, genre profilé 30, rideau 37 ou bras d'appui 40 peuvent constituer des équipements optionnels et être aménagés isolément ou en combinaison sur l'un ou l'autre des deux modes de réalisation décrits, ou sur des versions voisines.

L'orifice ouvert 12 peut éventuellement comporter une sorte de manchette de protection, similaire à la manchette 25 mais ouverte latéralement. D'autre part, le paravent peut comporter deux orifices circulaires pour le passage des bras ; dans ce cas, ces deux orifices seront avantageusement équipés de manchettes de protection 25.

## Revendications

1. Paravent en matériau radioprotecteur pour assurer la protection d'un opérateur contre les émissions de rayonnements ionisants du type rayons X ou autres, lequel paravent est constitué d'une paroi frontale (1) associée à une paroi latérale (2) s'étendant à l'équerre ou sensiblement à l'équerre à partir de l'un des côtés de ladite paroi frontale (1), lesquelles parois (1, 2) comportent des panneaux transparents (8, 10) sur une partie au moins de leur hauteur, **caractérisé en que** ladite paroi frontale (1) est constituée d'un panneau inférieur (7) vertical ou sensiblement vertical, prolongé par un panneau supérieur (8) dont une partie au moins est réalisée en matériau transparent, lequel panneau supérieur (8) est, d'une part, incliné vers l'avant, formant surplomb, faisant un angle compris entre 10° et 30° par rapport à la verticale, pour permettre audit opérateur de se rapprocher de la zone d'intervention, et d'autre part, muni de deux orifices (11, 12) pour le passage des bras dudit opérateur, et en ce que ladite paroi latérale (2) épouse la forme en dièdre de ladite paroi frontale (1).

2. Paravent de protection selon la revendication 1, **caractérisé en ce qu'**il comporte un panneau supérieur (8), incliné vers l'avant, faisant un angle compris entre 15 et 20° par rapport à la verticale.

3. Paravent de protection selon l'une quelconque des revendications 1 ou 2, **caractérisé en ce que** la paroi latérale (2) comporte un panneau inférieur opaque (9) et un panneau supérieur transparent (10) de forme sensiblement trapézoïdale, pour épouser la forme en dièdre de ladite paroi frontale (1).

4. Paravent de protection selon l'une quelconque des revendications 1 à 3, **caractérisé en ce que** la paroi paroi frontale (1) est constituée d'un panneau inférieur (7) en matériau opaque qui s'étend sur une hauteur comprise entre 60 et 100 cm, prolongé par un panneau supérieur (8) qui s'étend jusqu'à un niveau correspondant au moins à la taille de l'opérateur, c'est-à-dire 2 m.

5. Paravent de protection selon l'une quelconque des revendications 1 à 4, **caractérisé en ce que** l'un au moins des orifices (11, 12) de passage des bras de l'opérateur est doté d'une manchette (25) en matériau radioprotecteur.

6. Paravent de protection selon la revendication 5, **caractérisé en ce que** la manchette de protection (25) est constituée de lamelles souples (26) montées sur une couronne circulaire (27) qui est associable au rebord de l'orifice de réception, lesquelles lamelles (26) se chevauchent et sont maintenues par un bracelet souple (28) situé à proximité de l'embouchure externe de ladite manchette (25).

7. Paravent de protection selon l'une quelconque des revendications 1 à 6, **caractérisé en ce que** l'un des orifices (11) de passage de bras est situé à proximité de l'angle formé par les parois frontale (1) et latérale (2), l'autre orifice (12) étant situé sur le bord libre de ladite paroi frontale (1), ouvert latéralement.

8. Paravent de protection selon l'une quelconque des revendications 1 à 7, **caractérisé en ce que** le panneau frontal supérieur (8) muni des orifices (11, 12) de passage des bras est doublé au moyen d'un panneau mobile (13) muni desdits orifices (11 et 12) de passage des bras, lequel panneau frontal (8) comporte des orifices (11', 12') de forme oblongue, surdimensionnés par rapport auxdits orifices (11 et 12) dudit panneau mobile (13), qui s'étendent sur toute la surface balayée par lesdits orifices (11, 12) dudit panneau de doublage (13), notamment pour permettre un réglage de la hauteur desdits orifices (11, 12).

9. Paravent de protection selon la revendication 8, **caractérisé en ce que** le panneau mobile (13) coopère avec des moyens de guidage en forme de glissières (14, 15) fixées latéralement au niveau de la paroi frontale (1), et **en ce que** des moyens de verrouillage sont aménagés pour régler la position en hauteur dudit panneau mobile (13), lesquels moyens de verrouillage sont constitués d'un index (16) disposé sur le rebord de la paroi frontale (1) et d'un doigt d'ancrage (17) associé audit panneau mobile (13).

10. Paravent de protection selon l'une quelconque des revendications 1 à 7, **caractérisé en ce que** la paroi frontale (1) et la paroi latérale (2) forment un ensemble monté coulissant verticalement sur un bâti ou soubassement (4) muni de roulettes (5, 5'), notamment pour permettre un réglage de la hauteur des orifices (11, 12) de passage des bras.

11. Paravent de protection selon la revendication 10, **caractérisé en ce qu'**il comporte un système de manoeuvre de l'ensemble constitué par la paroi frontale (1) et la paroi latérale (2), en forme de vérin(s) piloté(s) par un organe de commande.

12. Paravent de protection selon l'une quelconque des revendications 1 à 11, **caractérisé en ce qu'**il comprend, solidaires de son bâti ou soubassement (4), des roulettes (5) placées au niveau des différentes arêtes, et au moins une roulette complémentaire (5') disposée en saillie sur la face avant de la paroi frontale (1), portée par une console (6), permettant d'accroître le périmètre de sustentation.

13. Paravent de protection selon l'une quelconque des revendications 1 à 12, **caractérisé en ce qu'**il comporte, fixées sur les parois frontale (1) et latérale (2) ou sur le châssis ou soubassement (4), des lamelles souples (32) en matériau du genre caoutchouc plombé.

14. Paravent de protection selon l'une quelconque des revendications 1 à 13, **caractérisé en ce qu'**il comporte, sur les faces externe et interne de la paroi frontale (1), sous le niveau des orifices (11 et 12) de passage des bras, des barrettes ou profilés (30) permettant notamment la fixation de champs stériles.

15. Paravent de protection selon l'une quelconque des revendications 1 à 14, **caractérisé en ce qu'**il comporte une paroi complémentaire (3) en matériau radioprotecteur, faisant office de plafond, qui s'étend entre les parois frontale (1) et latérale (2).

16. Paravent de protection selon l'une quelconque des revendications 1 à 15, **caractérisé en ce qu'**il comporte un rideau souple (37) de protection du dos de l'opérateur.

17. Paravent de protection selon l'une quelconque des revendications 1 à 16, **caractérisé en ce qu'**il comporte un bras d'appui (40) amovible de soutien de l'opérateur.

## Claims

1. A screen made of radioprotective material for ensuring protection of an operator against X ray-type ionising radiation emissions or others, which screen consists of a front wall (1) associated with a side wall (2) extending at right angle or substantially at right angle from one of the sides of said front wall (1), which walls (1, 2) include transparent panels (8, 10) over a portion at least of the height thereof, **characterised in that** said front wall (1) consists of a lower panel (7) vertical or substantially vertical, prolonged by an upper panel (8) whereof a portion at least is made of transparent material, whereof the upper panel (8), on the one hand is tilted forward, thereby overhanging, forming an angle ranging between 10° and 30° with respect to the vertical, allowing the operator to get closer to the operation area, and on the other hand, is fitted with two orifices (11, 12) for letting through said operator's arms, and **in that** said side wall (2) follows the dihedral shape of said front wall (1).

2. A protection screen according to claim 1, **characterised in that** it includes an upper panel (8), tilted forward, forming an angle ranging between 15 and 20° with respect to the vertical.

3. A protection screen according to any of the claims 1 or 2, **characterised in that** the side wall (2) includes an opaque lower panel (9) and a transparent upper panel (10) with a sensibly trapezoidal shape, to follow the dihedral shape of said front wall (1).

4. A protection screen according to any of the claims 1 to 3, **characterised in that** the front wall (1) is formed of a lower panel (7) of opaque material which extends over a height ranging between 60 and 100 cm, prolonged by an upper panel (8) which extends up to a level corresponding at least to the operator's height, i.e. 2 m.

5. A protection screen according to any of the claims 1 to 4, **characterised in that** one at least of the orifices (11, 12) for letting through the operator's arms is provided with an oversleeve (25) of radioprotective material.

6. A protection screen according to claim 5, **characterised in that** the protection oversleeve (25) is formed of flexible strips (26) mounted on a circular crown (27) which may be associated with the rim of the reception orifice, which strips (26) overlap and are maintained by a flexible wristband (28) situated close to the external mouthpiece of said oversleeve (25).

7. A protection screen according to any of the claims 1 to 6, **characterised in that** one of the orifices (11) for letting through the arms is situated close to the angle formed by the front (1) and lateral (2) walls, the other orifice (12) being situated on the free edge of said front wall (1), open laterally.

8. A protection screen according to any of the claims 1 to 7, **characterised in that** the upper front panel (8) fitted with orifices (11, 12) for letting through the arms is lined by dint of a mobile panel (13) fitted with said orifices (11 and 12) for letting through the arms, which front panel (8) includes orifices (11', 12') oblong in shape, oversized with respect to said orifices (11 and 12) of said mobile panel (13), which extend over the whole surface scanned by said orifices (11, 12) of said lining panel (13), notably for enabling adjustment in height of said orifices (11, 12).

9. A protection screen according to claim 8, **characterised in that** the mobile panel (13) co-operates with guiding means in the form of rails (14, 15) fixed laterally at the front wall (1), and **in that** locking means are arranged for adjusting the position in height of said mobile panel (13), which locking means are formed of an index (16) arranged on the rim of the front wall (1) and of an anchoring finger (17) associated with said mobile panel (13).

10. A protection screen according to any of the claims 1 to 7, **characterised in that** the front wall (1) and the side wall (2) form an assembly mounted to slide vertically on a frame or substructure (4) fitted with castor wheels (5, 5'), notably for enabling adjustment in height of the orifices (11,12) for letting through the arms.

11. A protection screen according to claim 10, **characterised in that** it includes a system for controlling the assembly composed of the front wall (1) and the side wall (2), in the form of actuator(s) driven by a control member.

12. A protection screen according to any of the claims 1 to 11, **characterised in that** it includes, integral with its frame or substructure (4), castor wheels (5) placed at the different ridges levels, and at least one additional castor wheel (5') mounted to protrude on the front face of the front wall (1), carried by a console (6), enabling to increase the sustentation perimeter.

13. A protection screen according to any of the claims 1 to 12, **characterised in that** it includes, attached to the front (1) and lateral (2) walls or to the chassis or substructure (4), flexible strips (32) made of leaded rubber-type material.

14. A protection screen according to any of the claims 1 to 13, **characterised in that** it includes, on the external and internal faces of the front wall (1), below the level of the orifices (11 and 12) for letting through the arms, small bars or profiles (30) enabling notably to attach sterile fields.

15. A protection screen according to any of the claims 1 to 14, **characterised in that** it includes an additional wall (3) of radioprotective material, acting as a ceiling, which extends between the front (1) and lateral (2) walls.

16. A protection screen according to any of the claims 1 to 15, **characterised in that** it includes a flexible curtain (37) for protecting the operator's back.

17. A protection screen according to any of the claims 1 to 16, **characterised in that** it includes a removable resting arm (40) for supporting the operator.

## Patentansprüche

1. Schirm aus Strahlenschutzmaterial zum Sicherstellen des Schutzes eines Bedieners vor den Emissionen von ionisierenden Strahlungen vom Typ Röntgenstrahlen oder anderen, wobei dieser Schirm von einer Stirnwand (1) gebildet wird, die mit einer Seitenwand (2) verbunden ist, die sich ausgehend von einer der Seiten der Stirnwand (1) rechtwinklig oder im Wesentlichen rechtwinklig zu dieser erstreckt, wobei die Wände (1, 2) auf wenigstens einem Abschnitt ihrer Höhe durchsichtige Platten (8, 10) aufweisen, **dadurch gekennzeichnet, dass** die Stirnwand (1) von einer vertikalen oder im Wesentlichen vertikalen unteren Platte (7) gebildet wird, die durch eine obere Platte (8) verlängert ist, von der wenigstens ein Abschnitt aus durchsichtigem Material hergestellt ist, wobei diese obere Platte (8) einerseits nach vorn geneigt ist, eine Auskragung bildend, wobei sie einen Winkel zwischen 10° und 30° bezüglich der Vertikalen aufweist, um dem Bediener zu ermöglichen, sich dem Eingriffsbereich zu nähern, und andererseits mit zwei Öffnungen (11, 12) zum Durchstecken der Arme des Bedieners ausgestattet ist, und **dadurch**, dass die Seitenwand (2) an die einem Dieder entsprechende Form der Stirnwand (1) angepasst ist.

2. Schutzschirm nach Anspruch 1, **dadurch gekennzeichnet, dass** er eine obere Platte (8) aufweist, die nach vorn geneigt ist, wobei sie einen Winkel zwischen 15° und 20° bezüglich der Vertikalen aufweist.

3. Schutzschirm nach einem der Ansprüche 1 oder 2, **dadurch gekennzeichnet, dass** die Seitenwand (2) eine undurchsichtige untere Platte (9) und eine durchsichtige obere Platte (10) von im Wesentlichen trapezförmiger Form aufweist, damit sie an die einem Dieder entsprechende Form der Stirnwand (1) angepasst ist.

4. Schutzschirm nach einem der Ansprüche 1 bis 3, **dadurch gekennzeichnet, dass** die Stirnwand (1) von einer unteren Platte (7) aus undurchsichtigem Material gebildet wird, welche sich auf einer Höhe zwischen 60 und 100 cm erstreckt, verlängert durch eine obere Platte (8), welche sich bis zu einer Höhe erstreckt, die wenigstens der Größe des Bedieners entspricht, das heißt 2 m.

5. Schutzschirm nach einem der Ansprüche 1 bis 4, **dadurch gekennzeichnet, dass** wenigstens eine der Öffnungen (11, 12) zum Durchstecken der Arme des Bedieners mit einer Manschette (25) aus Strahlenschutzmaterial versehen ist.

6. Schutzschirm nach Anspruch 5, **dadurch gekennzeichnet, dass** die Schutzmanschette (25) von flexiblen Lamellen (26) gebildet wird, die an einem kreisförmigen Kranz (27) angebracht sind, welcher mit dem Rand der Aufnahmeöffnung verbunden werden kann, wobei sich diese Lamellen (26) überlappen und von einem flexiblen Armreifen (28) gehalten werden, der sich in der Nähe der äußeren Mündung der Manschette (25) befindet.

7. Schutzschirm nach einem der Ansprüche 1 bis 6, **dadurch gekennzeichnet, dass** sich eine der Öffnungen (11) zum Durchstecken eines Arms in der Nähe des Winkels befinde der von der Stirnwand (1) und der Seitenwand (2) gebildet wird, während sich die andere Öffnung (12) an dem freien Rand der Vorderwand (1) befindet und seitlich offen ist.

8. Schutzschirm nach einem der Ansprüche 1 bis 7, **dadurch gekennzeichnet, dass** die obere Stirnplatte (8), die mit den Öffnungen (11, 12) zum Durchstecken der Arme ausgestattet ist, mittels einer beweglichen Platte (13) dubliert ist, die mit diesen Öffnungen (11 und 12) zum Durchstecken der Arme ausgestattet ist, wobei die Stirnplatte (8) Öffnungen (11', 12') von länglicher Form aufweist, die bezüglich der Öffnungen (1] und 12) der beweglichen Platte (13) überdimensioniert sind und die sich über die gesamte Fläche erstrecken, die von den Öffnungen (11, 12) der Dublierplatte (13) überstrichen wird, insbesondere um eine Einstellung der Höhe der Öffnungen (11, 12) zu ermöglichen.

9. Schutzschirm nach Anspruch 8, **dadurch gekennzeichnet, dass** die bewegliche Platte (13) mit Führungsmitteln in Form von Gleitschienen (14, 15) zusammenwirkt, die seitlich auf der Höhe der Stirnwand (1) befestigt sind, und **dadurch**, dass Verriegelungsmittel angebracht sind, um die Position der beweglichen Platte (13) in der Höhe einzustellen, wobei diese Verriegelungsmittel von einer Rastöffnungsreihe (16), die am Rand der Stirnwand (1) angeordnet ist, und einem Verankerungsstift (17), der mit der beweglichen Platte (13) verbunden ist, gebildet werden.

10. Schutzschirm nach einem der Ansprüche 1 bis 7, **dadurch gekennzeichnet, dass** die Stirnwand (1) und die Seitenwand (2) ein Ganzes bilden, das vertikal gleitend auf einem Untergestell oder Sockel (4) montiert ist, das bzw. der mit Laufrollen (5, 5') ausgestattet ist, insbesondere um eine Einstellung der Höhe der Öffnungen (11, 12) zum Durchstecken der Arme zu ermöglichen.

11. Schutzschirm nach Anspruch 10, **dadurch gekennzeichnet, dass** er ein System zur Betätigung des von der Stirnwand (1) und der Seitenwand (2) gebildeten Ganzen in Form einer Hubspindel (von Hubspindeln) aufweist, die durch eine Steuereinrichtung gesteuert wird (werden).

12. Schutzschirm nach einem der Ansprüche 1 bis 11, **dadurch gekennzeichnet, dass** er mit seinem Untergestell oder Sockel (4) fest verbundene Laufrollen (5) aufweist, die im Bereich der verschiedenen Kanten angebracht sind, und wenigstens eine zusätzliche Laufrolle (5'), die vorspringend an der Vorderseite der Stirnwand (1) angeordnet ist und von einer Konsole (6) getragen wird, die es ermöglicht, den Stützumfang zu vergrößern.

13. Schutzschirm nach einem der Ansprüche 1 bis 12, **dadurch gekennzeichnet, dass** er an der Stirnwand (1) und Seitenwand (2) oder an dem Gestell oder Sockel (4) befestigte flexible Lamellen (32) aus Material von der Art von Bleigummi aufweist.

14. Schutzschirm nach einem der Ansprüche 1 bis 13, **dadurch gekennzeichnet, dass** er an der Außenseite und Innenseite der Stirnwand (1) auf der Höhe der Öffnungen (1) und 12) zum Durchstecken der Arme Stege oder Profilteile (30) aufweist, die insbesondere die Befestigung von sterilen Feldern ermöglichen.

15. Schutzschirm nach einem der Ansprüche 1 bis 14, **dadurch gekennzeichnet, dass** er eine zusätzliche Wand (3) aus Strahlenschutzmaterial aufweist, die als obere Abdeckung dient, welche sich zwischen der Stirnwand (1) und der Seitenwand (2) erstreckt.

16. Schutzschirm nach einem der Ansprüche 1 bis 15, **dadurch gekennzeichnet, dass** er einen flexiblen Vorhang (37) zum Schutz des Rückens des Bedieners aufweist.

17. Schutzschirm nach einem der Ansprüche 1 bis 16, **dadurch gekennzeichnet, dass** er einen abnehmbaren Stützarm (40) zur Abstützung des Bedieners aufweist.
